# EUROPÄISCHE PATENTSCHRIFT

(11) **EP 2 234 996 B1**
(45) Veröffentlichungstag und Bekanntmachung des Hinweises auf die Patenterteilung: **28.08.2013**
(21) Anmeldenummer: 08863030.6
(22) Anmeldetag: 11.12.2008
(51) Int. Cl.: C07D 323/06

(54) **Verfahren zur Herstellung von Roh-Trioxan**
Method for the production of crude trioxane
Procédé de production de trioxane brut

(30) Priorität: 19.12.2007 EP 07150114
(43) Veröffentlichungstag der Anmeldung: 06.10.2010
(73) Patentinhaber: BASF SE, 67056 Ludwigshafen (DE)
(72) Erfinder: SIEGERT, Markus, 69126 Heidelberg (DE); LANG, Neven, 68163 Mannheim (DE); THIEL, Joachim, 67435 Neustadt (DE); STROEFER, Eckhard, 68163 Mannheim (DE); SIGWART, Christoph, 69469 Weinheim (DE)
(86) Internationale Anmeldenummer: PCT/EP2008/067287
(87) Internationale Veröffentlichungsnummer: WO 2009/077416

(56) Entgegenhaltungen:
- EP-A- 0 133 669
- DE-A1- 1 668 867
- DE-A1- 10 361 516
- DE-A1- 19 732 291
- DE-A1-102004 040 284
- DE-A1-102004 051 118

## Beschreibung

Die Erfindung betrifft ein Verfahren zur Herstellung von Roh-Trioxan mit einer Konzentration im Bereich von 50 bis 75 Gew.-% durch Trimerisieren von Formaldehyd aus einer wässrigen Formaldehyd-Lösung in Gegenwart eines sauren Katalysators und destillative Aufkonzentrierung des Trioxans aus dem Reaktionsgemisch der Trimerisierung.

Trioxan wird überwiegend durch Trimerisierung von Formaldehyd aus wässrigen Formaldehyd-Lösungen in homogener oder heterogener Katalyse in Gegenwart von sauren Katalysatoren erhalten. Problematisch ist hierbei, dass Formaldehyd zum Feststoffausfall, unter Bildung von Paraformaldehyd, neigt, wobei mit steigendem Formaldehydgehalt in einer wässrigen Lösung die Temperatur, bei der Feststoff auszufallen beginnt, abnimmt. Es gilt näherungsweise, zumindest für Formaldehydkonzentrationen im Bereich von 30 bis 70 Gew.-%, dass wässrige Formaldehyd-Lösungen auf eine Temperatur erhitzt werden müssen, die um etwa 10°C höher ist als die Konzentration an Formaldehyd in Gew.-%, um einen Feststoffausfall zu vermeiden. So muss eine 50-%ige wässrige Formaldehyd-Lösung auf etwa 60 bis 70°C erhitzt werden bzw. eine 60-%ige Formaldehyd-Lösung auf etwa 70 bis 80°C, um das Formaldehyd in Lösung zu halten.

Das durch Trimerisierung des Formaldehyds erhaltene Trioxan wird, gegebenenfalls gemeinsam mit weiteren Monomeren, überwiegend als Monomer zur Herstellung von Polyoxymethylenhomo- oder copolymeren eingesetzt.

Polymerisationsfähiges Trioxan muss bestimmten Spezifikationsanforderungen genügen und wird im Folgenden als Rein-Trioxan bezeichnet. Hierbei handelt es sich um einen Strom mit einem Mindestgehalt von 97,5 Gew.-% Trioxan, oder auch 99 Gew.-% Trioxan oder 99,5 Gew.-% Trioxan. Ein Strom mit einem Mindestgehalt von 99,9 Gew.-% Trioxan kann als Reinsttrioxan bezeichnet werden.

Die Gewinnung von Rein-Trioxan durch Aufkonzentrierung des Reaktionsgemisches der Trimerisierung von Formaldehyd ist verfahrenstechnisch dadurch erschwert, dass Trioxan, Formaldehyd und Wasser ein ternäres Azeotrop bilden, das bei einem Druck von 1 bar die Zusammensetzung 69,5 Gew.-% Trioxan, 5,4 Gew.-% Formaldehyd und 25,1 Gew.-% Wasser aufweist, und dessen Zusammensetzung stark druckabhängig ist, wobei der Trioxananteil bei niedrigeren Drücken höher ist.

Die Aufkonzentrierung des Trioxan/Formaldehyd/Wasser-Gemisches zu Rein-Trioxan wird daher technisch unter Umgehung der Azeotrop-Problematik beispielsweise durch Extraktion, wie in DE-A 1668867 beschrieben oder durch Pervaporation, wie in DE-A 197 32 291 beschrieben, durchgeführt. Diese Trennverfahren sind jedoch aufwendig durch die Kosten für Extraktionsmittel oder Pervaporationseinheit und die Handhabung derselben.

Daher wurde in DE 103 61 516 eine rektifikative Abtrennung durch Druckwechselrektifikation vorgeschlagen, wobei das TrioxanlFormaldehyd/Wasser-Gemisch aus der Trimerisierung von Formaldehyd in mehreren Kolonnen bei unterschiedlichen Drücken destillativ aufgetrennt wird. Hierbei wird in einer ersten Kolonne bei niedrigerem Druck Formaldehyd abgetrennt, d. h. ein im Wesentlichen trioxanfreier Formaldehyd/WasserStrom und ein trioxanhaltiger wässriger Strom mit wenig Formaldehyd erhalten. Dieser trioxanhaltige wässrige Strom mit wenig Formaldehyd wird in einer zweiten Kolonne, die bei höherem Druck gegenüber der ersten Kolonne betrieben wird, in reines Trioxan und ein Trioxan/Formaldehyd/Wasser-Gemisch mit niedrigerem Trioxan-Gehalt aufgetrennt, woraus in einer weiteren Kolonne im Wesentlichen reines Wasser abgetrennt wird und ein Trioxan/Formaldehyd/Wasser-Gemisch erhalten wird, das erneut in die erste Destillationskolonne recycliert wird.

Dieses Verfahren erfordert jedoch hohe Investitions- und Betriebskosten für die Vielzahl von Destillationskolonnen.

Es war demgegenüber Aufgabe der Erfindung, ein technisch einfaches Verfahren zur Verfügung zu stellen, wonach Roh-Trioxan mit einer möglichst hohen Trioxan-Konzentration durch Trimerisieren von Formaldehyd erhalten wird, so dass die weitere Aufkonzentrierung zu polymerisationsfähigem Rein-Trioxan weniger aufwändig ist.

Die Lösung besteht in einem Verfahren zur Herstellung von Roh-Trioxan mit einer Konzentration im Bereich von 50 bis 75 Gew.-% Trioxan, durch Trimerisieren von Formaldehyd aus einer wässrigen Formaldehyd-Lösung in Gegenwart eines sauren Katalysators und destillative Aufkonzentrierung des Trioxans aus dem Reaktionsgemisch der Trimerisierung, dadurch gekennzeichnet, dass
- die Trimerisierung des Formaldehyds und die Aufkonzentrierung des Trioxans aus dem Reaktionsgemisch der Trimerisierung in einer einzigen Kolonne durchgeführt werden, die
- durch ein horizontales Trennblech in einen unteren Kolonnenbereich A und einen oberen Kolonnenbereich B getrennt ist,
   wobei der untere Kolonnenbereich A und der obere Kolonnenbereich B über eine externe Dampf- und eine externe Flüssigkeitsleitung verbunden sind, und wobei
- im unteren Kolonnenbereich A bei einem Druck zwischen 1 und 5 bar eine Reaktivdestillation durchgeführt wird, wobei das Formaldehyd zu Trioxan trimerisiert und das Trioxan im Reaktionsgemisch bis zur Löslichkeitsgrenze des Formaldehyds aufkonzentriert wird, und
- im oberen Kolonnenbereich B bei einem Druck zwischen 200 und 900 mbar eine Destillation durchgeführt wird, wobei das Trioxan Roh-Trioxan mit einer Konzentration im Bereich von 50 bis 75 Gew.-% aufkonzentriert wird.

Es wurde gefunden, dass es möglich ist, Synthese und Aufkonzentrierung von Trioxan in einer einzigen Kolonne durchzuführen, die durch geeignete Wahl der Betriebsparameter Temperatur und Druck, abgestimmt auf das Löslichkeitsverhalten von Formaldehyd und Trioxan in wässrigen Gemischen, dergestalt betrieben wird, dass an keinem Punkt der Kolonne Feststoff ausfällt. Entsprechend ist die Anlagenverfügbarkeit erhöht.

Indem erfindungsgemäß ein Verfahren zur Verfügung gestellt wird, wonach Synthese und Aufkonzentrierung von Trioxan in einem einzigen Apparat durchgeführt werden können, sind die Investitionskosten gegenüber bekannten Verfahren niedriger.

Für die Trioxansynthese wird im Allgemeinen von einer wässrigen Formaldehyd-Lösung ausgegangen, die 45 bis 75 Gew.-% Formaldehyd, Rest Wasser, enthält. Diese Lösung kann, falls notwendig, in einem vorgelagerten Aufkonzentrierungsschritt aus einer wässrigen Formaldehyd-Lösung mit niedrigerer Formaldehyd-Konzentration erhalten werden. Der Aufkonzentrierungsschritt kann beispielsweise in einem Verdampfer, vorzugsweise einem Fallfilmverdamper, durchgeführt werden, wie beispielsweise in DE-A 199 25 870 beschrieben.

Die Trimerisierung des Formaldehyds wird in Gegenwart saurer, homogen oder heterogen vorliegender Katalysatoren, wie Ionenaustauscher, Harze, Zeolithe, Schwefelsäure oder Paratoluolsulfonsäure durchgeführt.

Die Synthese des Trioxans durch Trimerisierung von Formaldehyd erfolgt erfindungsgemäß im unteren Kolonnenbereich A einer Kolonne K, bei einem Druck zwischen 1 und 5 bar und einer diesem Druck entsprechenden Temperatur im Bereich von etwa 100 bis 200°C.

Durch Trimerisieren des Formaldehyds entsteht ein Trioxan enthaltendes Reaktionsgemisch, das im unteren Kolonnenbereich, an trennwirksamen Einbauten, rektifikativ an Trioxan aufkonzentriert wird. Die Anzahl der theoretischen Trennstufen im unteren Kolonnenbereich wird im Bereich von etwa 1 bis 20 dergestalt ausgelegt, dass das Reaktionsgemisch am oberen Ende des unteren Kolonnenbereichs so hoch wie möglich an Trioxan aufkonzentriert wird, wobei jedoch gewährleistet sein muss, dass der aus dem oberen Ende des unteren Kolonnenbereichs abgezogene Brüdenstrom nach Einleitung über eine externe Leitung in den unteren Bereich des oberen Kolonnenbereichs, der bei niedrigerem Druck gegenüber dem unteren Kolonnenbereich betrieben wird, nicht zu Feststoffausfall führt.

Die Kolonne ist mittels eines horizontalen Trennblechs in einen unteren Kolonnenbereich und einen oberen Kolonnenbereich flüssigkeits- und gasdicht getrennt. Das obere Ende des unteren Kolonnenbereichs und das untere Ende des oberen Kolonnenbereichs sind jeweils über eine externe, außerhalb der Kolonne angeordnete Leitung miteinander verbunden.

Im oberen Kolonnenbereich findet eine weitere Aufkonzentrierung an Trioxan unter atmosphärischem Druck, im Bereich zwischen 200 und 900 mbar, statt. Weiter bevorzugt wird die destillative Aufkonzentrierung bei einem Druck zwischen 500 und 700 mbar durchgeführt.

Die Anzahl der theoretischen Trennstufen im Destillationsteil der Kolonne, d. h. im oberen Kolonnenbereich, wird dergestalt ausgelegt, dass am Kolonnenkopf möglichst hochkonzentriertes Roh-Trioxan abgezogen werden kann, wobei jedoch gewährleistet sein muss, dass an keiner Stelle in der Kolonne Feststoff ausfällt. Die Zahl der theoretischen Trennstufen im oberen Kolonnenbereich kann vorzugsweise von 1 bis 25 betragen.

Vorteilhaft kann aus dem oberen oder unteren Kolonnenbereich ein wasserhaltiger Strom flüssig oder gasförmig abgezogen und in eine nachfolgende Verfahrensstufe recycliert werden.

Am Kolonnenkopf wird ein Roh-Trioxan-Strom abgezogen, enthaltend 50 bis 75 Gew.-% Trioxan, oder auch 60 bis 75 Gew.-% Trioxan.

Die Erfindung wird im Folgenden anhand eines Ausführungsbeispiels und einer Zeichnung näher erläutert.

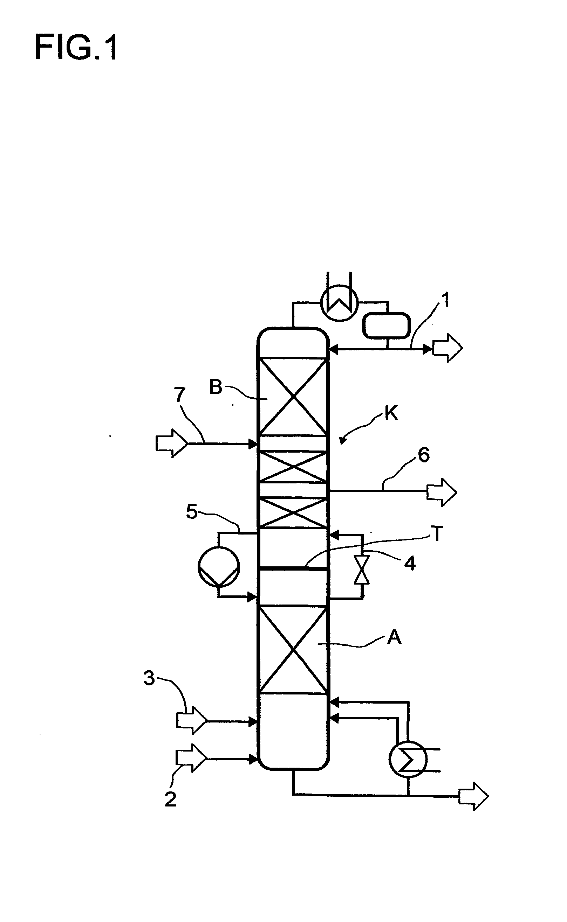

Die einzige Figur 1 zeigt eine Kolonne K mit horizontaler Trennwand T, die die Kolonne in einen unteren Kolonnenbereich A und einen oberen Kolonnenbereich B aufteilt.

Am Kolonnenkopf wird Roh-Trioxan, Strom 1, abgezogen. Im unteren Teil des unteren Kolonnenbereichs A wird eine wässrige Formaldehyd-Lösung, Strom 2, und saurer Katalysator-Strom 3 zugeführt. Der untere Kolonnenbereich A ist mit dem oberen KoIonnenbereich B über eine externe Dampf- und eine externe Flüssigkeitsleitung 5 verbunden.

In der in der Figur dargestellten bevorzugten Ausführungsform wird aus dem oberen Kolonnenbereich B ein wasserhaltiger Strom 6 flüssig abgezogen.

Bevorzugt kann, wie in der Figur dargestellt, in den oberen Kolonnenbereich B ein trioxan- und/oder formaldehydhaltiger Strom 7 zugeführt werden.

## Patentansprüche

1. Verfahren zur Herstellung von Roh-Trioxan (1) mit einer Konzentration im Bereich von 50 bis 75 Gew.-% Trioxan, durch Trimerisieren von Formaldehyd aus einer wässrigen Formaldehyd-Lösung (2) in Gegenwart eines sauren Katalysators (3) und destillative Aufkonzentrierung des Trioxans aus dem Reaktionsgemisch der Trimerisierung, **dadurch gekennzeichnet, dass**
- die Trimerisierung des Formaldehyds und die Aufkonzentrierung des Trioxans aus dem Reaktionsgemisch der Trimerisierung einer einzigen Kolonne (K) durchgeführt werden, die
- durch ein horizontales Trennblech (T) in einen unteren Kolonnenbereich (A) und einen oberen Kolonnenbereich (B) getrennt ist,
wobei der untere Kolonnenbereich (A) und der obere Kolonnenbereich (B) über eine externe Dampf- und eine externe Flüssigkeitsleitung verbunden sind, und wobei
- im unteren Kolonnenbereich (A) bei einem Druck zwischen 1 und 5 bar eine Reaktivdestillation durchgeführt wird, wobei das Formaldehyd zu Trioxan trimerisiert und das Trioxan im Reaktionsgemisch bis zur Löslichkeitsgrenze des Formaldehyds aufkonzentriert wird, und
- im oberen Kolonnenbereich (B) bei einem Druck zwischen 200 und 900 mbar eine Destillation durchgeführt wird, wobei das Trioxan zu Roh-Trioxan mit einer Konzentration im Bereich von 50 bis 75 Gew.-% aufkonzentriert wird.

2. Verfahren nach Anspruch 1, **dadurch gekennzeichnet, dass** Roh-Trioxan mit einer Konzentration im Bereich von 60 bis 75 Gew.-% hergestellt wird.

3. Verfahren nach Anspruch 1 oder 2, **dadurch gekennzeichnet, dass** die Reaktivdestillation im unteren Kolonnenbereich (A) bei einem Druck zwischen 1,25 und 1,75 bar und die Destillation im oberen Kolonnenbereich (B) bei einem Druck im Bereich von 500 bis 700 mbar durchgeführt wird.

4. Verfahren nach einem der Ansprüche 1 bis 3, **dadurch gekennzeichnet, dass** im unteren Kolonnenbereich (A) 1 bis 20 theoretische Trennstufen und im oberen Kolonnenbereich (B) 1 bis 25 theoretische Trennstufen vorgesehen sind.

5. Verfahren nach einem der Ansprüche 1 bis 5, **dadurch gekennzeichnet, dass** aus dem oberen Kolonnenbereich (B) oder dem unteren Kolonnenbereich (A) ein wasserhaltiger, flüssiger oder gasförmiger Strom abgezogen und in eine nachgeschaltete Verfahrensstufe recycliert wird.

## Claims

1. A process for preparing crude trioxane (1) having a concentration in the range from 50 to 75% by weight of trioxane by trimerization of formaldehyde from an aqueous formaldehyde solution (2) in the presence of an acid catalyst (3) and concentration of the trioxane from the reaction mixture from the trimerization by distillation,
wherein
- the trimerization of the formaldehyde and the concentration of the trioxane from the reaction mixture from the trimerization are carried out in a single column (K) which
- is divided by a horizontal dividing plate (T) into a lower column region (A) and an upper column region (B),
with the lower column region (A) and the upper column region (B) being connected by an external vapor line and an external liquid line, and
- a reactive distillation in which the formaldehyde is trimerized to trioxane and the trioxane in the reaction mixture is concentrated to the solubility limit of formaldehyde is carried out in the lower column region (A) at a pressure in the range from 1 to 5 bar and
- a distillation in which the trioxane is concentrated to give crude trioxane having a concentration in the range from 50 to 75% by weight is carried out in the upper column region (B) at a pressure in the range from 200 to 900 mbar.

2. The process according to claim 1, wherein crude trioxane having a concentration in the range from 60 to 75% by weight is prepared.

3. The process according to claim 1 or 2, wherein the reactive distillation is carried out in the lower column region (A) at a pressure in the range from 1.25 to 1.75 bar and the distillation in the upper column region (B) is carried out at a pressure in the range from 500 to 700 mbar.

4. The process according to any of claims 1 to 3, wherein from 1 to 20 theoretical plates are provided in the lower column region (A) and from 1 to 25 theoretical plates are provided in the upper column region (B).

5. The process according to any of claims 1 to 4, wherein a water-comprising, liquid or gaseous stream is taken off from the upper column region (B) or the lower column region (A) and is recycled to a downstream process stage

## Revendications

1. Procédé de fabrication de trioxane brut (1) présentant une concentration dans la plage allant de 50 à 75 % en poids de trioxane, par trimérisation de formaldéhyde à partir d'une solution aqueuse de formaldéhyde (2) en présence d'un catalyseur acide (3) et concentration distillative du trioxane à partir du mélange réactionnel de la trimérisation, **caractérisé en ce que**
- la trimérisation du formaldéhyde et la concentration du trioxane à partir du mélange réactionnel de la trimérisation sont réalisées dans une colonne (K) unique, qui
- est divisée par une plaque de séparation horizontale (T) en une zone de colonne inférieure (A) et une zone de colonne supérieure (B),
la zone de colonne inférieure (A) et la zone de colonne supérieure (B) étant reliées par une conduite de vapeur externe et une conduite de liquide externe, et
- une distillation réactive étant réalisée dans la zone de colonne inférieure (A) à une pression comprise entre 1 et 5 bar, le formaldéhyde étant trimérisé en trioxane et le trioxane étant concentré dans le mélange réactionnel jusqu'à la limite de solubilité du formaldéhyde, et
- une distillation étant réalisée dans la zone de colonne supérieure (B) à une pression comprise entre 200 et 900 mbar, le trioxane étant concentré en trioxane brut présentant une concentration dans la plage allant de 50 à 75 % en poids.

2. Procédé selon la revendication 1, **caractérisé en ce que** le trioxane brut est fabriqué en une concentration dans la plage allant de 60 à 75 % en poids.

3. Procédé selon la revendication 1 ou 2, **caractérisé en ce que** la distillation réactive dans la zone de colonne inférieure (A) est réalisée à une pression comprise entre 1,25 et 1,75 bar et la distillation dans la zone de colonne supérieure (B) à une pression dans la plage allant de 500 à 700 mbar.

4. Procédé selon l'une quelconque des revendications 1 à 3, **caractérisé en ce que** 1 à 20 étapes de séparation théoriques sont prévues dans la zone de colonne inférieure (A) et 1 à 25 étapes de séparation théoriques dans la zone de colonne supérieure (B).

5. Procédé selon l'une quelconque des revendications 1 à 4, **caractérisé en ce qu'**un courant aqueux, liquide ou gazeux, est soutiré de la zone de colonne supérieure (B) ou de la zone de colonne inférieure (A) et recyclé dans une étape de procédé en aval.
